# EUROPEAN PATENT SPECIFICATION

(11) **EP 1 479 380 B1**
(45) Date of publication and mention of the grant of the patent: **09.05.2007**
(21) Application number: 03011028.2
(22) Date of filing: 19.05.2003
(51) Int. Cl.: A61K 9/20, A61K 9/16, A61K 31/522

(54) **Use of soaps such as magnesium stearate for increasing the release of drugs**
Verwendung von Seifen wie Magnesium Stearate zur Beschleunigung der Freisetzung von Wirkstoffen
Utilisation de savons tel que le magnesium stearate pour accélérer la libération des substances actives

(43) Date of publication of application: 24.11.2004
(73) Proprietor: EURO-CELTIQUE S.A., 2330 Luxembourg (LU)
(72) Inventor: Rein, Hubert, 35396 Giessen-Wieseck (DE); Steffens, Klaus-Jürgen, 53359 Rheinbach-Flerzheim (DE)
(74) Representative: Maiwald Patentanwalts GmbH

(56) References cited:
- WO-A-03/028703
- DE-A- 19 916 383
- FR-A- 1 279 907
- FR-A- 1 397 102
- RU-C- 2 188 000
- US-A- 3 957 662
- US-A- 5 679 685

## Description

The present invention concerns the use of soaps for increasing the release of pharmaceutical active compounds from matrix-based pharmaceutical dosage forms. The present invention also concerns methods for producing fast release matrix-based pharmaceutical dosage forms wherein the pharmaceutical dosage forms are formulated with soaps. Moreover, the invention concerns matrix-based pharmaceutical dosage forms wherein the dosage forms comprise a soap in an amount sufficient to ensure a fast release of a pharmaceutically active compound from the dosage form.

Matrix-based pharmaceutical dosage forms are among the most common dosage forms in pharmaceutical technology. They are usually produced by molding processes such as extrusion. Production of dosage forms by extrusion has the advantage that the dosage forms can be obtained in a continuous process which often is more time- and cost-efficient than production of comparable dosage forms by e.g. granulation.

However, since the matrix of such pharmaceutical dosage forms usually comprises pharmaceutically acceptable polymers, production of such dosage forms by extrusion encounters many technical problems. These problems include clogging of the extruder's nozzle, exposure the pharmaceutically active compound to non-acceptable friction heat blocking of the extruder due to the high viscosity of polymers etc.

To circumvent these problems, the matrix-forming polymers and the pharmaceutically active compounds are usually co-extruded with common, pharmaceutically acceptable excipients. Those excipients include e.g. lubricants, such as waxes, fatty alcohols, plasticizers such as dibutyl sebacate, slipping agents such as aerosils etc.

Another feature of matrix-based pharmaceutical dosage forms obtained by extrusion is that they often provide for an extended or sustained release of the pharmaceutically active compound. In many cases, sustained-released dosage forms may be the desired product.

However, there is also a need for matrix-based pharmaceutical dosage forms which may be produced by extrusion and provide for a fast or rapid release. The advantage of production by extrusion of such dosage forms is that production by extrusion is more time-and cost-efficient. Another advantage of such dosage forms is that the pharmaceutically active compound(s) usually can hardly or only with significant difficulties be extracted from the dosage forms. This aspect becomes particularly relevant when pharmaceutically active compounds are used that have an addictive potential and therefore cannot be used in prescription-free, over-the-counter products. Typical examples for pharmaceutically active compounds where a dosage form is necessary from which the active compound should be extractable only with significant difficulties, are analgesics such as opiates or opioids. Matrix-based pharmaceutical dosage forms which provide for a fast release of the active compound are moreover important e.g. for pain therapy where sudden pain attacks have to be counteracted in a fast and efficient way.

A general drawback of common extruded pharmaceutical dosage forms derives from the use of matrix-forming compounds such as plastics, waxes, fatty alcohols, thermoplastics and duroplastics which are not subject to biological degradation. One way to circumvent this problem may be the use of biological-degradable matrix-forming polymers such as cellulose or starch. Dosage forms with starch matrices have been disclosed in WO 00/64415.

However, matrix-based pharmaceutical dosage forms which provide for the above-mentioned characteristics are not known in the art. Thus there is a strong need for matrix-based pharmaceutical dosage forms which allow for a substantially rapid release of the pharmaceutically active compound while at the same time ensuring that the compound can hardly, or not at all, be extracted from the dosage forms. Moreover, there is a strong need for such dosage forms which additionally comprise a matrix which is bio-degradable and are storage stable.

It is therefore an objective of the present invention to provide matrix-based pharmaceutical dosage forms which allow for a rapid release of the pharmaceutically active ingredient. It is another objective of the present invention to provide for such pharmaceutical dosage forms from which the active compound can be extracted only with significant difficulties and which preferably comprise a matrix that is biological degradable. Another objective of the present invention is to provide pharmaceutical dosage forms with the above-mentioned characteristics which can be produced by means of extrusion.

These and other objectives of the present invention, as may become clear from the description, are obtained by the features of the independent claims. Preferred embodiments of the present invention are defined by the dependent claims.

Above-mentioned International patent application WO 00/64415 describes the production of pharmaceutical dosage forms which comprise a starch matrix, by extrusion. According to WO 00/64415 sustained release pharmaceutical dosage forms comprising a starch matrix may be produced by extrusion of the starch polymers together with a metal soap, such as magnesium stearate (Mg-Stearate).

Mg-Stearate is well known in the art as a typical lubricant for extrusion processes. Moreover, it is well known that Mg-Stearate can also be used to delay the release of pharmaceutically active compounds from pharmaceutical dosage forms. US 3,728,445 discloses that metal salts such as Mg-Stearate may be used as a barrier to delay penetration of fluids into the center of the tablet. By this barrier function, the metal soaps ensure that the pharmaceutically active compounds are released in a delayed fashion from the pharmaceutical dosage form.

In the context of the present invention is has now surprisingly been found that in certain amounts soaps and preferably metals soaps such as Mg-Stearate may be used in order to produce matrix-based pharmaceutical dosage forms which show an increased or fast release of the pharmaceutically active compound from the dosage form, instead of providing the slow release known in the art.

Without wanting to be bound to any scientific theory, it is assumed that the release-enhancing effect of soaps and particularly Mg-Stearate at the amounts disclosed by the present invention is due to creation of "defective" sites within the matrices. In the case of polymer-based matrices such as starch matrices, it is thus assumed that Mg-Stearate reduces polymer-polymer interactions, thereby "loosening" the polymer meshwork and allowing the compound to be released more rapidly. At Mg-Stearate concentrations higher than those disclosed by the invention, the known physical-chemical characteristics of Mg-Stearate become predominant, thus providing for the sustained-release effect well known in the art.

One object of the present invention is therefore the use of soaps and preferably of Mg-Stearate for increasing the release of pharmaceutically active compounds from matrix-based pharmaceutical dosage forms compared to dosage forms produced without the respective metal soap.

The present invention also concerns the use of soaps and preferably of metals soaps such as Mg-Stearate for producing matrix-based pharmaceutical dosage forms with a substantially fast release of the pharmaceutically active compound.

Another object of the present invention concerns a method of increasing the release of pharmaceutically active compounds from matrix-based pharmaceutical dosage forms wherein the pharmaceutical dosage form is formulated with soaps and preferably with metals soaps such as Mg-Stearate.

Yet another object of the present invention concerns matrix-based pharmaceutical dosage forms wherein the dosage form comprises a soap in an amount sufficient to ensure a substantially fast release of a pharmaceutically active compound from the dosage forms. Such dosage forms provide for a good storage stability.

The use of soaps and preferably of metals soaps such as Mg-Stearate may have additional advantages for the final product. Extruded formulations may show a more uniform diameter over the whole extruded product when Mg-Stearate is used. Moreover, the tendency for die-swell and temperature inhomogenities may be reduced due to the use of Mg-Stearate. In some cases matrix-forming polymers such as certain starch types may be efficiently extrudable only upon inclusion of Mg-Stearate.

In the context of the present invention the term "soap" comprises metal soaps and alkali soaps.

In the context of the present invention the term "metal soap" relates to the salts of multivalent metals and higher fatty acids. Thus, the term "metal soap" preferably relates to the AI, Ba, Ca, Cd, Co, Cr, Cu, Fe, Li, Mg, Mn, Ni, Pb, Sn, Sr, Zn salts of fatty acids. According to the present invention fatty acids comprise acids such as stearic acid, palmitic acid, oleic acid or linoleic acid. A particularly preferred metal soap in the context of the present invention is Mg-Stearate.

In the context of the present invention the term "alkali soap" relates to the salts of alkali metals and higher fatty acids. Thus, the term "alkali soap" preferably relates to the Li, Na, K, Rb and Cs salts of fatty acids. According to the present invention fatty acids comprise acids such as stearic acid, palmitic acid, oleic acid or linoleic acid.

In the context of the present invention the term "pharmaceutically active compound" relates to all common pharmaceutically active compounds which may be formulated into matrix-based dosage forms by common processes such as granulation or extrusion. Pharmaceutically active compounds according to the present invention thus comprise antipyretic, analgesic and anti-inflammatory agents such as indomethacin, aspirin, diclofenac, ibuprofren, antiulcer agents such as sulpiride, coronary vasodilaters such as nifedipine, peripheral vasodilators such as ifenprodil tartrate, antibiotics such as ampicillin, chloramphenicol or erythromycin, synthetic antimicrobial agents such as nalidixic acid, antispasmodic agents such as propantheline bromide, antitussive and antiasthmatic agents such as theophylline or aminophylline, bronchodilators such as diprophylline, diuretics such as furosemide, muscle relaxants such as chlorophenesin carbamate, cerebral metabolism improving agents such meclofenoxate hydrochloride, minor tranquilizers such as oxazolam, diazepam or clotiazepam, major tranquilizers such as sulpiride, beta-blockers such as pindolol, antiarrhythmic agents such as procainamide hydrochloride, anticoagulants such as ticlopidine hydrochloride, antiepileptics such as phenytoin, antihistaminics such as chlorpheniramine maleate, antiemetics such as difenidol hydrochloride, antihypertensive agents such dimethylaminoethyl reserpilinate hydrochloride, sympathomimetic agents such as dihydroergotamine mesilate, expectorants such as bromhexine hydrochloride, oral antidiabetic agents such as glibenclamide, cardiovascular system drugs such as ubidecarenone, iron preparations such as ferrous sulfate, non stearoidal anti-inflammatory drugs or vitamins. Particularly preferred are analgesics comprising the group of opiates and opioides such as oxycodone, morphine, dihydrocodeine, hydromorphine, oxymorphine, buprenorphine or tramadol. Also preferred are analgesic antagonists such as naltrexone or naloxone. Other opioid agonists and antagonists can be found, e.g. in WO 99/32119.

Even though this might not be expressly stated, the term "active compound" always comprises pharmaceutical acceptable and equally acting derivatives, salts and the like. If, for example, naloxone is mentioned, this also comprises, besides the free base, its hydrochloride, sulfate, bisulfate, tatrate, nitrate, citrate, bitatrate, phosphate, malate, maleate, hydrobromide, hydrojodide, fumarate, succinate and the like.

When in the context of the present invention, the term "increased release" is used in connection with a matrix-based dosage form, this refers to the fact that matrix-based dosage forms in accordance with the invention provide a faster release due to the use of soaps within the preparation than those preparations that are formulated without the soaps but are otherwise substantially identical with respect to their composition.

When in the context of the present invention the term "substantially fast release" is used in connection with a matrix-based pharmaceutical dosage form this refers to the fact that matrix-based dosage forms in accordance with the invention do not provide a sustained release as common sustained-release formulations do.

Preferably, these preparations will have substantially released all of the active compounds after 12, preferably after 10, more preferably after 8, even more preferably after 6 and most preferably after 4 hours. According to the present invention a matrix-based pharmaceutical dosage form provides for a fast release of the pharmaceutically active compound if at least 50 % of the compound is released after 3 hours, preferably after 2 hours, more preferably after 1 hour, even more preferably after 30 minutes, and most preferably after 15 minutes.

According to the present invention a matrix-based pharmaceutical preparation also provides for a fast release if the preparation releases at least 75 % of the active compound after 8, 7 or 6 hours, preferably after 5 hours, more preferably after 4 hours, even more preferably after 2 hours, also even more preferably after 1 hour, and most preferably after 30 minutes.

According to the present invention, a matrix-based pharmaceutical dosage form is considered to be "hardly" extractable if the pharmaceutically active compound can be (mechanically) extracted from the pharmaceutical dosage form only with significant difficulties. Thus dosage forms in accordance with the invention may be comminutable only upon usage of tools like a hammer. Hardly extractable pharmaceutical dosage forms according to the present invention are moreover characterised by matrix stability towards acids, bases and comparable solvents. A starch matrix according to the invention e.g. neither dissolves in the acidic environment of the stomach nor in the neutral environment of the intestine.

According to the invention "storage stable" or "storage stability" means that upon storage under standard conditions (at least two years at room temperature and usual humidity) the amounts of the active compounds of a medicament formulation do not deviate from the initial amounts by more than the values given in the specification or the guidelines of the common Pharmacopoeias. According to the invention, storage stability also means that a preparation produced according to the invention can be stored under standard conditions (60 % relative humidity, 25 °C) as it is required for admission to the market.

Release rates of active compounds from preparations in accordance with the invention are measured according to the Pharmacopeia Europeae (Pharm. Eur.). Release of active compounds is typically measured either in 0.1 nHCl pH 1.0 or Sorensen-buffer pH 6.8. Measurement was performed either with HPLC or by UV-Vis-Spectroscopy (see also Rein et al. (2001) UV-Vis-Spectroscopy, Deutsche Apothekerzeitung, 26, 53 ff).

According to the present invention the term "matrix-based pharmaceutical dosage forms" comprises those dosage forms that comprise the pharmaceutically active compound within a matrix in the sense as known to the person skilled in the art. The matrix-based pharmaceutical dosage forms may be coated additionally with layers as e.g. enteric coatings. Such layers may be formed by typical film layer forming polymers such as polymers of the commercially available Eudragit® - or Surelease® -type and may additionally influence the release behaviour of the dosage forms. They may also comprise an initial amount of the pharmaceutically active compound as well as typical pharmaceutical excipients such as colorants, odorants and conserving agents.

According to the present invention, the soaps are used at a concentration of 0.5 to 3.5 % (w/w), preferably at a 1 to 3% (w/w), more preferably at a 1.5 to 2.5% (w/w) and most preferably at approximately 2% (w/w) referred to the weight of the complete dosage form.

According to the present invention, the metal soaps are used at a concentration of 0.5 to 3.5 % (w/w), preferably at a 1 to 3% (w/w), more preferably at a 1.5 to 2.5% (w/w) and most preferably at approximately 2% (w/w) referred to the weight of the complete dosage form.

According to the present invention, the matrix-based dosage forms may be produced by common pharmaceutical techniques such as granulation or extrusion. Production by extrusion is a preferred embodiment of the present invention.

Extrusion may be performed as it is known in the art. Thus, extrusion may be performed using a single-screw extruder or a twin-screw extruder. Twin-screw extruders may comprise either counter- or co-rotating screws with or without kneading elements. Preferred extruder types for single-screw and twin-screw extruders are given in the example section. The exact extrusion parameters can be easily determined by the person skilled in the art. They depend on the extruder type, the pharmaceutically active compound, the material used for preparation of the matrix as well as the additional pharmaceutical excipients. Extrusion parameters comprise parameters such as the heating temperatures for the different extruder zones, the nozzle diameter and form, the rotating speed of the screw, the water content of the preparation as well as the order of addition of the different components. Preferred embodiments for specific extruders are given in the example section.

According to the present invention the matrix of the matrix-based pharmaceutical dosage form comprises a matrix-forming polymer, a soap, at least one pharmaceutically active compound and optionally pharmaceutically acceptable excipients.

The pharmaceutically acceptable excipients comprise lubricants, plasticizers, colorants, conserving agents, viscosity-influencing agents, surfactants, fillers etc.. Pharmaceutically acceptable excipients may therefore comprise lactose as filler, surfactants such as sodium lauryl sulfate or silicium dioxide, hydrophilic or amphiphilic liquids such as polyglycols, glycerol, polyethylene glycol, fatty acids, fats, saturated carbohydrates, etc.

The water content of the final, dried extruded preparations is typically below 15%, preferably below 10%. However, the preparations may be extruded at significantly higher water contents such as e.g. 20%, 25%, 30% or even more than 35%. They may also be processed at lower water contents, such 15 %, 10 % or 7,5 % water. All percentages indicate the amount of water based on the total weight of the preparation.

The matrix-forming polymers may be preferably of a polysaccharide type including common polymers such as cellulose, cellulose derivatives or starch. According to the invention the use of starch for forming the matrix is preferred. Especially preferred is the use of starches such as tapioca-, potato-, corn- or wheat-starch. Commercially available starches such as starch 1500® (a corn-starch distributed by Colorcon), Waxylis® (a corn-starch distributed by Roquette) or Eurylon7® (a corn-starch distributed by Roquette) are preferred. The use of corn starch is particularly preferred. However, other starch types such as acetyl-starch may also be used. The above-mentioned starch or derivatives thereof may also be mixed for forming a matrix-based pharmaceutical dosage form according to the invention. If there is no need for the matrix to consist of biological degradable polymers, other matrix-forming polymers such as polyvinyl pyrrolidone, methacrylic polymers and other matrix-forming polymers or substances such as waxes which are well known in the art may be used.

According to the present invention the matrix of the pharmaceutical dosage form comprises a water-insoluble and non-erosive diffusion matrix.

According to the present invention the pharmaceutically active compounds which are embedded in the matrix are released from the matrix substantially according to the lapidus-function.

In particularly preferred embodiments of the invention matrix-based pharmaceutical dosage forms are produced by extrusion of starch together with the pharmaceutically active compounds, soap(s) and optionally pharmaceutically acceptable excipients. The preparation of starch matrixes by extrusion comprising a pharmaceutically active compound has been described in detail in WO 00/64415 which is hereby explicitly incorporated as reference.

During the extrusion process, i.e. while using or applying heat, shear forces and pressure, a preferably amorphous or partially amorphous matrix is generated from the crystalline or partly crystalline polysaccharide or mixtures thereof.

If starch is extruded together with a pharmaceutically active compound, a soap and optionally pharmaceutically acceptable excipients, an amorphous or partly amorphous matrix is obtained.

When extruding starch, heating usually is only necessary at the beginning of the process. In the further course of the process, the heat generated by the strong shear and friction is preferably eliminated by cooling, in order to maintain a constant temperature. The formulations used according to the invention are comprised of a mixture of polysaccharides and/or derivatives and preferably of a mixture of starch and/or starch derivatives there being various types of starch which are suitable. Furthermore, the mixture contains at least one pharmaceutically active compound in an amount of up to 50 %, preferably of up to 30 %, more preferably of up to 20 %, and even more preferably of up to 10 % based on the total weight of the dosage form.

After thoroughly mixing and the addition of water the obtained pre-blend should preferably be screened so that it is free of lumps, in order to ensure perfect conveyance through the screw-feeder. Starting and cleaning of the extruder may for example be carried out using corn grit. When generating a matrix according to the invention, the temperature at the orifices of the extruder should not exceed 100°C under normal pressure, since with temperatures above 100°C the formation of a matrix free of pores will hardly be possible. The total energy fed into the process, in the form of shear forces, temperature, heat or pressure should be as constant as possible and should be sufficient to achieve glass transition temperature. The optimal screw speed and geometry of dies may be adjusted to the mixture comprised of the starch, an active compound, the soaps and optional pharmaceutically excipients. At extrusion temperatures below 100 °C and suitable screw speeds, transparent and completely amorphous products are obtained in most cases. The degree of the plastification of the active compound/polysaccharide mixture and preferably of the active agent/starch mixture is closely linked to the screw speed. While extrusion is no longer possible below the lower speed, a screw speed which is too high causes the dosage form to "pop open".

Generally, parameters that lead to "popped" products should be avoided, as so-called "popped" or "foamed" products do not allow for production of matrix-based pharmaceutical dosage forms with a reproducible release behaviour.

Dosage forms with rapid or substantially fast release may preferably be obtained for example by extrusion below the temperature of gelatinisation of the polysaccharides and preferably of the starches used in each case.

The person skilled in the art is aware that parameters determined for extrusion of a particular starch type on a specific extruder may have to be adapted if different starch types or extruders are used.

The dosage forms produced according to the present invention may be used to produce granulates for tabletting and filling capsules, and adjuvants for direct tabletting, for further processing by means of extrusion molding techniques and/or as mono-block dosage forms, where the extrudate is formed by means of suitable arrangement at the extrusion die - similar to the case of soap production. The invention allows the production and use of mono-block forms, in the process of which an extrudate is created that is vitrified only at the surface and includes the active compounds/carrier mixture in an unchanged state in its interior. In order to achieve the desired bio-pharmaceutical properties of the dosage form, co-adjuvants known for producing solid forms may be used.

In the following examples are given for the production of matrix-based pharmaceutical dosage forms with an increased and/or fast release of the pharmaceutically active compound due to the use of a soap. The preparations are produced by extrusion of different starch types together with caffeine as a model pharmaceutically active substance and Mg-Stearate as a metal soap either in a single screw or twin screw extruder.

The examples are meant to illustrate the invention and should by no means be interpreted in a restrictive manner.

### 1. Extrusion with a single-screw extruder

For production of the extruded products, starch, the pharmaceutically active compound and Mg-Stearate were mixed in a Stephan®-mixer for 15 minutes and extruded in a single-screw Brabender-extruder. The amount of Mg-Stearate was 2 % based on the weight of the dosage forms. The following parameters were used during the extrusion process:

Extrusion may be performed with a single-screw extruder. One particularly preferred extruder is the Brabender extruder Type 811201 (Brabender, Duisburg, Germany). The screw of this extruder does not comprise compression elements and the extruder may be heated in three different zones, particularly at the feeder, the barrel and at the die segment. The screw may be run at speeds from 25 to 250 revolutions per minute (rpm) and the feeding rate may be 20 to 250 g/minute. The nozzles may provide for a diameter of 1 to 10 mm, preferably of 2 to 8 mm and more preferably of 3 to 5 mm.

Using a single screw extruder such as the Brabender extruder, the extrusion process can be separated into multiple steps such as starting of the extrusion process, the extrusion process itself, removal of remaining compound-containing extruded material in the barrel, cleaning of the die segment and the barrel.

The extrusion process may preferably be started with wet corn grit in order to avoid to high torque Once the extruder has reached a stable state the compound-containing starch mixture together with Mg-Stearate may be added. The removal of remaining compound containing extruded material as well as the cleaning of the die segment and the barrel may be performed as it is known in the art.

Preferably, the screw speed during the extrusion process is between 90 to 150 rpm, more preferably between 100 and 130 rpm, and most preferably around 100, 110 or 120 rpm. The temperature at the feeder is typically between 30 to 100°C, preferably between 40 to 90°C, more preferably between 50 to 70°C, and even more preferably around 55, 60 and 65°C. The heating temperature at the barrels may be between 30 to 120°C, preferably between 40 to 90°C, more preferably between 50 to 80°C, and even more preferably approximately around 55, 60, 65, 70, 75 or 80°C. The temperature at the nozzle may be preferably between 80 to 120°C, more preferably between 95 to 100°C, and most preferably around 98 to 100°C. The nozzle diameter preferably is 2.5, 3.5 or 5 mm.

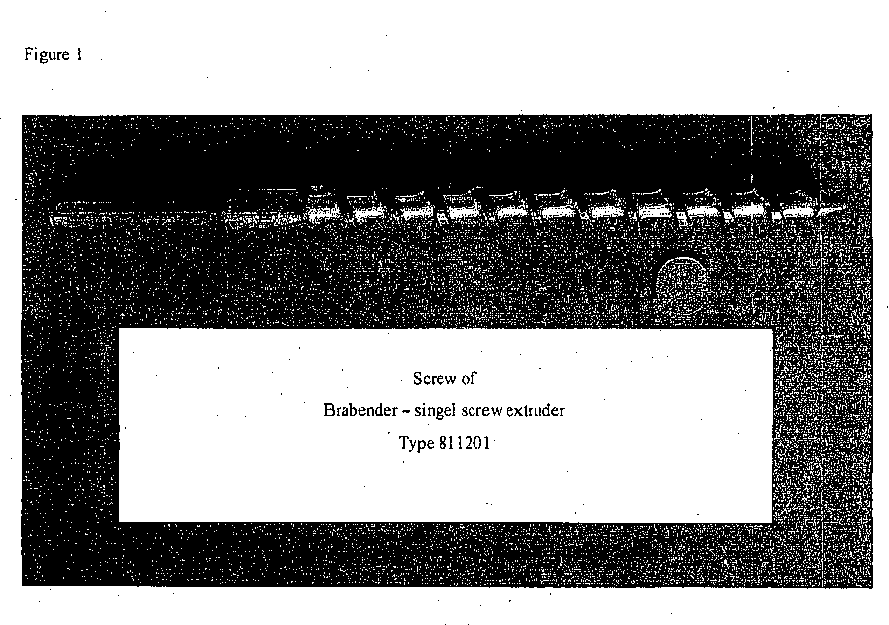

The geometry of the Brabender-extruder screw is shown in figure 1.

### 1.1. Extrusion of potato-starch

According to the above-specified parameters, a matrix-based pharmaceutical dosage form was extruded on basis of potato-starch as the matrix-forming material and caffeine as the pharmaceutically active compound.

The preparations were extruded at a screw speed of 120 rpm. The temperature at the feeder was 66°C, the temperature at the nozzle was 98°C.

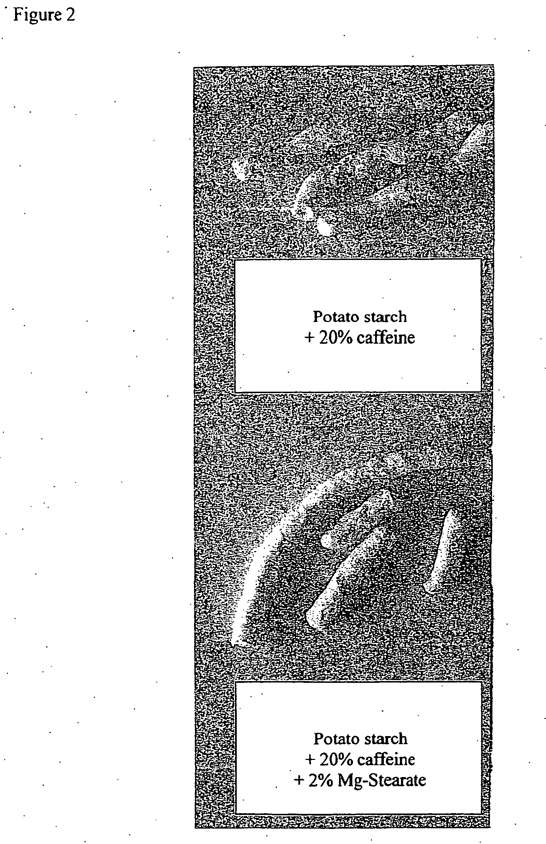

Figure 2 shows the outer appearance of pharmaceutically active compound-free dosage forms and dosage forms comprising 20 % caffeine with or without 2 % Mg-Stearate. All percentages are by weights based on the weight of the dosage form.

Addition of Mg-Stearate leads to a significantly improved appearance of the product. The surface of the Mg-Stearate-containing extrudate is smooth. The die swell is constant over the whole production process so that the extrudate comprises a uniform diameter. While Mg-Stearate-free extrudates of potato-starch typically show bulges, these are missing for the Mg-Stearate-containing extrudates. The tendency for melt fracture and temperature inhomogenities is significantly reduced for Mg-Stearate-containing extrudates compared to Mg-Stearate-free extrudates.

The release rates were measured either in Sörensen-buffer pH 6.8 or 0.1 n HCl pH 1.0 according to the European Pharmacopoeia paddle test as outlined above.

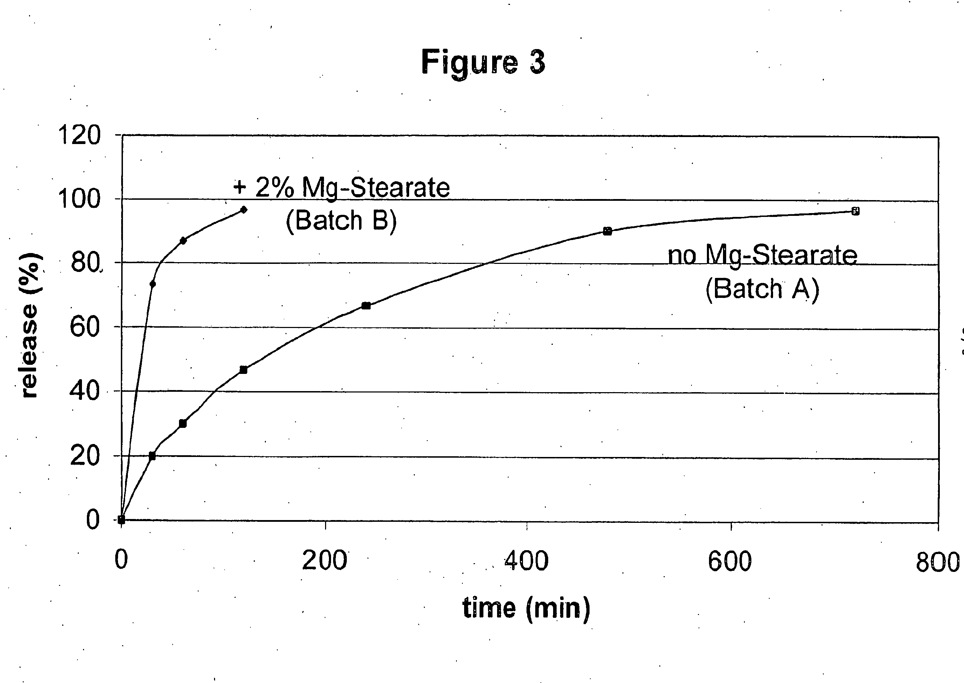

Figure 3 shows the release rates of the caffeine-potato-starch-extrudates. Each curve represents five measurements. The release rates are shown in comparison to those of a Mg-Stearate-free extrudate in table 1.

**Table I**

| Batch | Mg-Stearate [ % ] | Release | | | | | | | |
|---|---|---|---|---|---|---|---|---|---|
| | | of 50% after | of 75% after | 0,5h | 1 h | 2 h | 4 h | 8 h | 12 h |
| | | [min] | | [%] | | | | | |
| A | - | 134 | 302 | 20 | 30 | 46.7 | 66.7 | 90 | 96.7 |
| B | 2,0 | 16 | 34 | 73.3 | 86.7 | 96.7 | | | |

From figure 3 and table 1 one can clearly see that the addition of 2% (w/w) Mg-Stearate results in a significantly increased release rate of caffeine from the preparation compared to the Mg-Stearate-free product.

For production of batch A the screw-barrel was cooled. For production of batch C, the temperature at the feeder was 68 C, the temperature at the screw-barrel was 82 °C and the temperature at the noozle was 98 %. The screw speed was 120 rpm.

### 1.2. Extrusion of tapioca-starch

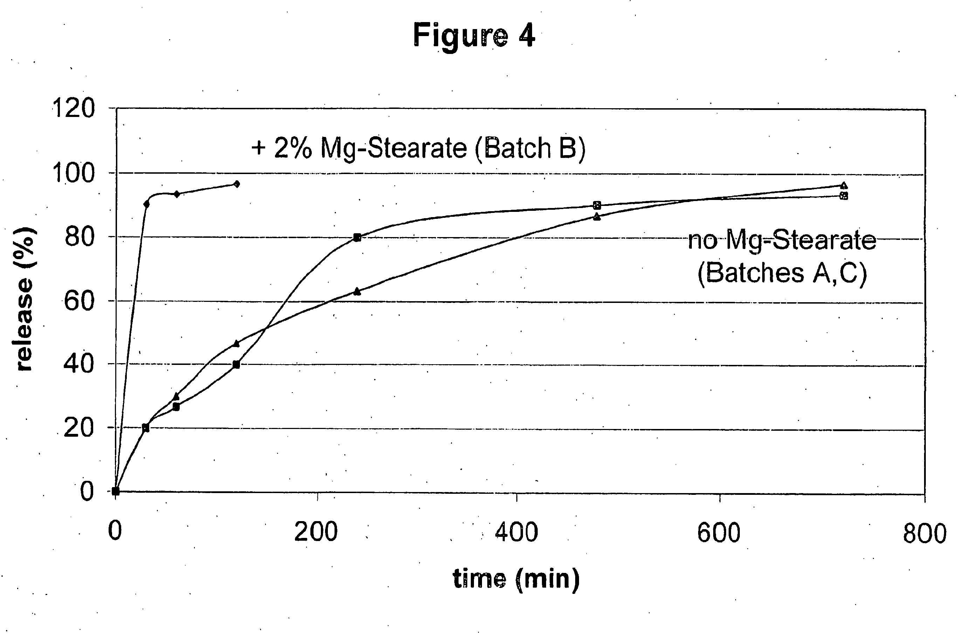

Tapioca-starch was extruded with 20 % caffeine and 2 % Mg-Stearate at the above-mentioned parameters. For batches A and B the temperature at the feeder was 64°C, the temperature at the nozzle was 99°C. The screw speed was 100 rpm. The release parameters were again measured according to the Pharm. Eur. paddle method in 0.1 n HCl pH 1.0 or Sörensen-buffer pH 6.8. For production of batch A the screw-barrel was cooled. For production of batch C, the temperature at the feeder was 68°C, the temperature at the screw-barrel was 82°C and the temperature at the noozle was 98°C. The screw speed was 120 rpm. The release data are shown in table 2 and figure 4.

**Table 2**

| Batch | Mg-Stearate [ % ] | Release | | | | | | | |
|---|---|---|---|---|---|---|---|---|---|
| | | of 50% after | of 75% after | 0,5h | 1 h | 2 h | 4 h | 8 h | 12 h |
| | | [min] | | [%] | | | | | |
| A | - | 178 | 413 | 20 | 26.7 | 40 | 53.3 | 80 | 93.3 |
| B | 2,0 | 8 | 16 | 90 | 93.3 | 96.7 | | | |
| C | - | 145 | 339 | 20 | 30 | 46.7 | 63.3 | 86.7 | 96.7 |

One clearly can infer from figure 4 and table 2 that the addition of 2 % Mg-Stearate significantly changes the release behaviour of caffeine from sustained release to fast release.

### 2. Extrusion with a twin-screw extruder

For the following experiments different starch types were extruded with a twin-screw extruder. For extrusion with twin screw extruders the following parameters may be used:

During extrusion with twin-screw-extruders, screw speeds may be between 150 to 250 rpm, preferably between 170 to 230 rpm, more preferably between 190 to 210 rpm, and most preferably around 200 rpm.

The temperature at the feeder may be between 20 to 80°C, preferably between 30 to 60°C, more preferably between 40 to 50°C, and even more preferably around 40, 45 or 50°C.

The temperature in the barrel heating zones may be between 30 to 180°C, between 40 to 160°C, between 60 to 140°C and between 80 to 120°C. Temperatures of approximately 40, 50, 60, 70, 75, 80, 115, 120, 125, 150, 155, 160°C are also preferred. Typically, the temperature of the heating zones of the barrel which are close to the nozzle will be lower than the temperature of those heating zones which are close to the feeder.

The temperature at the nozzle may be between 20 to 80°C, preferably between 30 to 60°C, and even more preferably around 30, 40 or 50°C.

### 2.1 Extrusion of corn-starch

White-corn-starch (available from Cerestar) was extruded together with 10 % caffeine and 2 % Mg-Stearate using a Werner & Pfleiderer ZSK 25 twin-screw extruder at the following parameters:

| | |
|---|---|
| Screw speed: | 202 rpm |
| Delivery rate of the screw: | 87.20 g/min |
| Water content: | 12.1 % |

| Temperature profile: | |
|---|---|
| Feeder: | 40°C |
| Barrel: | 120 / 155 / 75 / 40°C |
| Nozzle: | 30°C |

The white-corn starch could efficiently be extruded only when Mg-Stearate was added. Otherwise, the extruder would get blocked.

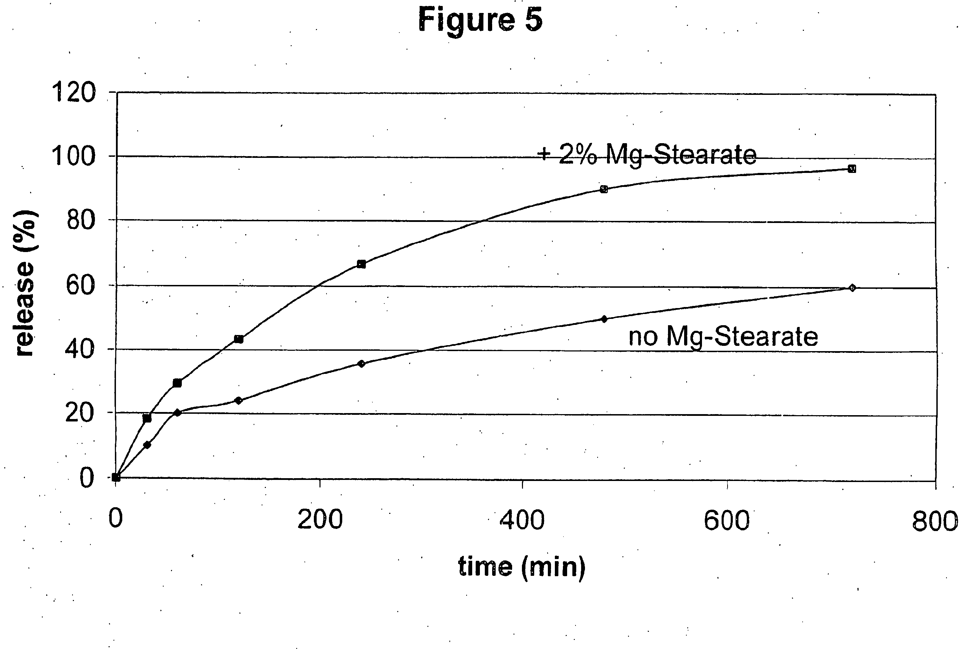

Figure 5 shows the release parameters for different batches with or without 2 % Mg-Stearate-containing extrudates. It can be clearly seen that the addition of 2 % Mg-Stearate results in a significantly increased release of caffeine from the extrudates.

The release rates were again measured according to the Pharm. Eur. paddle method in 0.1 n HCl pH 1.0 or Sörensen-buffer pH 6.8.

### 2.2 Extrusion of potato-starch

For the following experiment the potato starch C*Gel 30002, (available from Cerestar) was extruded at the following parameters:

| | |
|---|---|
| Screw speed: | 200 rpm |
| screw delivery rate: | 76.6 g/min for 10 % caffeine |
| | 69.3 g/min for 30 % caffeine |
| Water content: | 11-22% |

### Temperature profile for 10 % caffeine:

| | |
|---|---|
| Feeder: | 49°C |
| Barrels 1-4: | 123 / 154 / 76 / 52°C |
| Nozzle: | 52°C |

### Temperature profile for 30 % caffeine:

| | |
|---|---|
| Feeder: | 40°C |
| Barrel: | 120 / 155 / 76 / 26°C |
| Nozzle: | 22°C |

Potato-starch - caffeine mixtures were extruded at a caffeine-content of 10 - 30 % (w/w) based on the weight of the preparation. The addition of Mg-Stearate was 2 % (w/w) based on the weight of the preparation. As with the extrusion of white-corn starch, extrusion is easily performed upon addition of Mg-Stearate.

All potato-starch extrudates showed a significant increase of the release rates for caffeine if they contained 2 % Mg-Stearate compared to compositions that contained no Mg-Stearate.

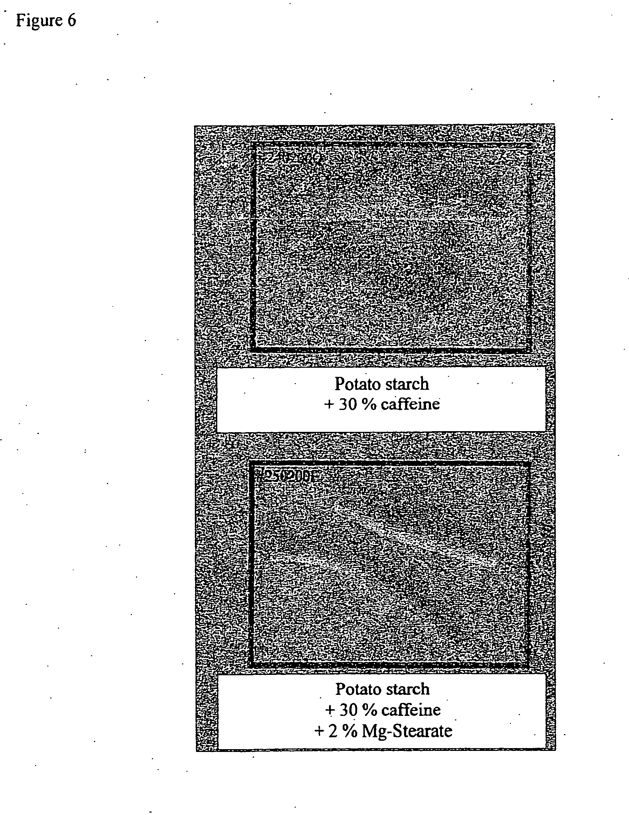

The outer appearance of the potato-starch extrudates is shown in figure 6.

Addition of Mg-Stearate leads to a significantly improved appearance of the product. The surface of the Mg-Stearate-containing extrudate is smooth. The die swell is constant over the whole production process so that the extrudate comprises a uniform diameter. While Mg-Stearate-free extrudates of potato-starch typically show bulges, these are missing for the Mg-Stearate-containing extrudates. The tendency for melt fracture and temperature inhomogenities is significantly reduced for Mg-Stearate-containing extrudates compared to Mg-Stearate-free extrudates.

Release data for caffeine-containing potato-starch extrudates with or without Mg-Stearate were obtained as described above. Addition of Mg-Stearate to the preparation led to a significant increase in the release of caffeine from the preparation.

### 2.3. Extrusion of tapioca-starch

In a last set of experiments tapioca-starch of the C-type crystal form (available from Thai Wah Public, Rose Brand) was extruded at the following parameters:

| | |
|---|---|
| Screw speed: | 200 to 400 rpm |
| screw delivery rate: | 86.50 g/min |
| Water content: | 14 - 18 % |

| Temperature profile: | |
|---|---|
| Feeder: | 40°C |
| Barrel: | 120 / 155 / 75 / 40°C |
| Nozzle: | 30°C |

Preparations were formulated with 10 % or 30 % caffeine (w/w) based on the weight of the preparation. Release rates were analysed by the Pharm. Eur. paddle method at 0.1 nHCl pH 1.0 or Sörensen-buffer pH 6.8 for the release of caffeine.

Addition of Mg-Stearate to the preparation led to a significant increase in the release of caffeine from the preparation.

Taken together the results of the above-described experiments can be summarized as follows. Independent of the extrusion method (single-screw or twin-screw extruder), of the starch type and the crystal form of the starch type, it can be observed that the addition of a soap such as Mg-Stearate allows for the production of matrix-based pharmaceutical dosage forms with increased release rates compared to those dosage forms which do not contain soaps. In view of the characteristics attributed to Mg-Stearate by the prior art this was completely unexpected and allows the person skilled in the art to formulate matrix-based, pharmaceutical dosage forms with predictable and reproducible release behaviours. This is particular an advantage when such dosage forms are produced by extrusion where it is known that the extrusion parameters may significantly influence the release behaviour in an often unpredictable way.

The surprising effect of Mg-Stearate on the release behaviour of pharmaceutically active compounds from extruded starch matrix dosage forms may be explained such that Mg-Stearate at the above-identified concentrations functions as a breaking point or a void-forming substance in the polymeric starch matrix. While Mg-Stearate-free dosage forms homogenously swell over the whole release period, Mg-Stearate-containing extrudates seem to disintegrate into multiple subunits with a significantly increased surface which is responsible for the increased release rate of the active compound.

A second effect of the use of Mg-Stearate is that the viscosity of the extruded melt is decreased. In this way Mg-Stearate may be used for efficiently producing matrix-based pharmaceutical dosage forms by extrusion which at the same time release the active compound in a fast or increased manner compared to preparations which comprise no Mg-Stearate. This applies particularly when starch is used during extrusion as the matrix forming material.

## Claims

1. Use of soaps for providing matrix-based pharmaceutical dosage forms with increased and/or fast-release of pharmaceutically active compounds from the matrix-based dosage form.

2. Use of soaps according to claim 1,
**characterized in that**, at least one alkali soap, preferably one or more alkali salts of higher fatty acids and more preferably the Li, Na, K salts of Stearate, Palmitate, Oleate or Linoleate are used as the soaps.

3. Use of soaps according to claim 1,
**characterized in that**, that at least one metal soap, preferably one or more metal salts of higher fatty acids and more preferably the Al, Ba, Ca, Cd, Co, Cr, Cu, Fe, Li, Mg, Mn, Ni, Pb, Sn, Sr, Zn salts of Stearate, Palmitate, Oleate or Linoleate are used as the soaps.

4. Use of metal soaps according to claim 3,
**characterized in that**, Mg-Stearate is used.

5. Use of soaps according to one of claims 1 to 4,
**characterized in that,** the soaps are used at 0.5 to 3.5% (w/w), preferably at 1 to 3% (w/w), more preferably at 1.5 to 2.5 % (w/w) and most preferably at approximately 2% (w/w) based on the weight of the dosage form.

6. Use of soaps according to one of claims 1 to 5,
**characterized in that**, the matrix-based dosage form is produced by extrusion.

7. Use of soaps according to one of claims 1 to 6,
**characterized in that**, the matrix comprises a matrix-forming polymer, at least one pharmaceutically active compound, a soap and optionally pharmaceutically acceptable excipients.

8. Use of soaps according to one of claims 1 to 7,
**characterized in that**, the matrix is a diffusion matrix.

9. Use of soaps according to claim 8,
**characterized in that**, the matrix-forming polymer is a polysaccharide, preferably from the group comprising cellulose, cellulose-derivatives or starch.

10. Use of soaps according to claim 9,
**characterized in that**, the matrix-forming polymer is starch, preferably potato-, tapioca- or com-starch.

11. Method of increasing the release rate of pharmaceutically active compounds from matrix-based pharmaceutical dosage forms, said release rate is measured according to the Pharm. Eur. paddle method,
**characterized in that,** the pharmaceutical dosage form is formulated with at least one soap component providing an increased and/or fast-release of the pharmaceutically active compounds from the matrix-based pharmaceutical dosage forms.

12. Method according to claim 11,
**characterized in that**, soaps according to one of claims I to 10 are used.

13. Matrix-based pharmaceutical dosage form,
**characterized in that**, the dosage form comprises at least one soap for providing an increased and/or fast-release of a pharmaceutically active compound from the dosage form in an amount sufficient to ensure an increased and/or fast release of a pharmaceutically active compound from the dosage form.

14. Matrix-based pharmaceutical dosage form according to claim 13,
**characterized in that**, at least one alkali soap, preferably one or more of the alkali salts of higher fatty acids and more preferably the Li, Na, K salts of Stearate, Palmitate, Oleate or Linoleate are used.

15. Matrix-based pharmaceutical dosage form according to claim 13,
**characterized in that**, at least one metal soap, preferably one or more metal salts of higher fatty acids and more preferably the Al, Ba, Ca, Cd, Co, Cr, Cu, Fe, Li, Mg, Mn, Ni, Pb, Sn, Sr, Zn salts of Stearate, Palmitate, Oleate or Linoleate are used.

16. Matrix-based pharmaceutical dosage form according to claim 15,
**characterized in that,** Mg-Stearate is used as the metal soap.

17. Matrix-based pharmaceutical dosage form according to one of claim 13 to 16,
**characterized in that,** the soap is used at 0.5 to3.5% (w/w), preferably at 1 to 3% (w/w),
more preferably at 1.5 to 2.5 % (w/w) and most preferably at approximately 2% (w/w) based on the weight of the dosage form.

18. Matrix-based pharmaceutical dosage form according to one of claims 13 to 17,
**characterized in that**, the matrix-based dosage form is produced by extrusion.

19. Matrix-based pharmaceutical dosage form according to one of claims 13 to 18,
**characterized in that**, the matrix comprises a matrix-forming polymer, a pharmaceutically active compound, a soap and optionally pharmaceutically acceptable excipients.

20. Matrix-based pharmaceutical dosage form according to one of claims 13 to 19,
**characterized in tha**t, the matrix is a diffusion matrix.

21. Matrix-based pharmaceutical dosage form according to one of claims 13 to 20,
**characterized in that**, the matrix-forming polymer is a polysaccharide selected from the group comprising cellulose, cellulose-derivatives and starch.

22. Matrix-based pharmaceutical dosage form according to claim 21,
**characterized in that**, the matrix-forming polymer is starch, preferably potato-, tapioca- or com-starch.

## Patentansprüche

1. Verwendung von Seifen zur Bereitstellung von Matrix-basierten pharmazeutischen Dosierungsformen mit einer erhöhten und/oder schnellen Freisetzung der pharmazeutisch aktiven Verbindungen aus der Matrix-basierten Dosierungsform.

2. Verwendung von Seifen gemäß Anspruch 1,
**dadurch gekennzeichnet, dass** mindestens eine Alkaliseife, bevorzugt ein oder mehrere Alkalisalze höherer Fettsäuren und noch bevorzugter die Li-, Na- oder K-Salze von Stearat, Palmitat, Oleat oder Linoleat als Seifen verwendet werden.

3. Verwendung von Seifen gemäß Anspruch 1,
**dadurch gekennzeichnet, dass** mindestens eine Metallseife, bevorzugt ein oder mehrere Metallsalze höherer Fettsäuren und noch bevorzugter die Al-, Ba-, Ca-, Cd-, Co-, Cr-, Cu-, Fe-, Li-, Mg-, Mn-, Ni-, Pb-, Sn-, Sr- oder Zn-Salze von Stearat, Palmitat, Oleat oder Linoleat als Seifen verwendet werden.

4. Verwendung von Metallseifen gemäß Anspruch 3,
**dadurch gekennzeichnet, dass** Mg-Stearat verwendet wird.

5. Verwendung von Seifen gemäß einem der Ansprüche 1 bis 4,
**dadurch gekennzeichnet, dass** die Seifen in einer Konzentration von 0,5 to 3,5 Gew.-%, bevorzugt von 1 bis 3 Gew.-%, bevorzugter von 1,5 bis 2,5 Gew.-% und am meisten bevorzugt von ca. 2 Gew.-%, basierend auf dem Gewicht der Dosierungsform, verwendet werden.

6. Verwendung von Seifen gemäß einem der Ansprüche 1 bis 5,
**dadurch gekennzeichnet, dass** die Matrix-basierte Dosierungsform durch Extrusion hergestellt wird.

7. Verwendung von Seifen gemäß einem der Ansprüche 1 bis 6,
**dadurch gekennzeichnet, dass** die Matrix ein Matrix-bildendes Polymer, mindestens einen pharmazeutisch aktiven Wirkstoff, eine Seife und optional pharmazeutisch verträgliche Hilfsstoffe umfasst.

8. Verwendung von Seifen gemäß einem der Ansprüche 1 bis 7,
**dadurch gekennzeichnet, dass** es sich bei der Matrix um eine Diffusionsmatrix handelt.

9. Verwendung von Seifen gemäß Anspruch 8,
**dadurch gekennzeichnet, dass** das Matrix-bildende Polymer ein Polysaccharid, bevorzugt aus der Gruppe umfassend Cellulose, Cellulose-Derivate oder Stärke ist.

10. Verwendung von Seifen gemäß Anspruch 9,
**dadurch gekennzeichnet, dass** das Matrix-bildende Polymer Stärke und bevorzugt Kartoffel-, Maniok- oder Maisstärke ist.

11. Verfahren zur Erhöhung der Freisetzungsgeschwindigkeit von pharmazeutisch aktiven Verbindungen aus Matrix-basierten pharmazeutischen Dosierungsformen, wobei die Feisetzungsgeschwindigkeit gemäß der Pharm. Eur. Rührblattmethode gemessen wird,
**dadurch gekennzeichnet, dass** die pharmazeutische Dosierungsform mit mindestens einer Seifenkomponente formuliert wird, die eine erhöhte und/oder schnelle Freisetzung der pharmazeutisch aktiven Wirkstoffe aus der Matrix-basierten pharmazeutischen Dosierungsform gewährleistet.

12. Verfahren gemäß Anspruch 11,
**dadurch gekennzeichnet, dass** Seifen gemäß einem der Ansprüche 1 bis 10 verwendet werden.

13. Matrix-basierte pharmazeutische Dosierungsform,
**dadurch gekennzeichnet, dass** die Dosierungsform mindestens eine Seife zum Gewährleisten einer erhöhten und/oder schnellen Freisetzung des pharmazeutisch aktiven Wirkstoffs aus der Dosierungsform in einer Menge umfasst, die ausreichend ist, um eine erhöhte und/oder schnelle Freisetzung der pharmazeutisch aktiven Verbindung aus der Dosierungsform sicherzustellen.

14. Matrix-basierte pharmazeutische Dosierungsform gemäß Anspruch 13,
**dadurch gekennzeichnet, dass** mindestens eine Alkaliseife, bevorzugt ein oder mehrere Alkalisalze höherer Fettsäuren und insbesondere bevorzugt die Li-, Na- oder K-Salze von Stearat, Palmitat, Oleat oder Linoleat verwendet werden.

15. Matrix-basierte pharmazeutische Dosierungsform gemäß Anspruch 13,
**dadurch gekennzeichnet, dass** mindestens eine Metallseife, bevorzugt ein oder mehrere Metallsalze höherer Fettsäuren und besonders bevorzugt die Al-, Ba-, Ca-, Cd-, Co-, Cr-, Cu-, Fe-, Li-, Mg-, Mn-, Ni-, Pb-, Sn-, Sr- oder Zn-Salze von Stearat, Palmitat, Oleat oder Linoleat verwendet werden.

16. Matrix-basierte pharmazeutische Dosierungsform gemäß Anspruch 15,
**dadurch gekennzeichnet, dass** Mg-Stearat als Metallseife verwendet wird.

17. Matrix-basierte pharmazeutische Dosierungsform gemäß einem der Ansprüche 13 bis 16,
**dadurch gekennzeichnet, dass** die Seife in einer Konzentration von 0,5 bis 3 Gew.-%, bevorzugt von 1 bis 3 Gew.-%, bevorzugter von 1,5 bis 2,5 Gew.-% und am meisten bevorzugt von ca. 2 Gew.-%, basierend auf dem Gewicht der Dosierungsform, verwendet wird.

18. Matrix-basierte pharmazeutische Dosierungsform gemäß einem der Ansprüche 13 bis 17,
**dadurch gekennzeichnet, dass** die Matrix-basierte Dosierungsform durch Extrusion hergestellt wird.

19. Matrix-basierte pharmazeutische Dosierungsform gemäß einem der Ansprüche 13 bis 18,
**dadurch gekennzeichnet, dass** die Matrix ein Matrix-bildendes Polymer, eine pharmazeutisch aktive Verbindung, eine Seife und optional pharmazeutisch verträglich Hilfsstoffe umfasst.

20. Matrix-basierte pharmazeutische Dosierungsform gemäß einem der Ansprüche 13 bis 19,
**dadurch gekennzeichnet, dass** es sich bei der Matrix um eine Diffusionsmatrix handelt.

21. Matrix-basierte pharmazeutische Dosierungsform gemäß einem der Ansprüche 13 bis 20,
**dadurch gekennzeichnet, dass** das Matrix-bildende Polymer ein Polysaccharid, ausgewählt aus der Gruppe umfassend Cellulose, Cellulose-Derivate oder Stärke ist.

22. Matrix-basierte pharmazeutische Dosierungsform gemäß Anspruch 21,
**dadurch gekennzeichnet, dass** das Matrix-bildende Polymer Stärke und bevorzugt Kartoffel-, Maniok-, oder Maisstärke ist.

## Revendications

1. Utilisation de savons pour fournir des formes posologiques pharmaceutiques matricielles présentant une libération accrue et/ou rapide de composés pharmaceutiquement actifs à partir de la forme posologique matricielle.

2. Utilisation de savons selon la revendication 1, **caractérisée en ce que** au moins un savon alcalin, de préférence un ou plusieurs sels alcalins d'acides gras supérieurs et de préférence les sels de Stéarate, Palmitate, Oléate ou Linoléate de Li, Na, K sont employés en tant que savons.

3. Utilisation de savons selon la revendication 1, **caractérisée en ce que** au moins un savon métallique, de préférence un ou plusieurs sels métalliques d'acides gras supérieurs et de préférence les sels de Stéarate, Palmitate, Oléate ou Linoléate de Al, Ba, Ca, Cd, Co, Cr, Cu, Fe, Li, Mg, Mn, Ni, Pb, Sn, Sr, Zn sont employés en tant que savons.

4. Utilisation de savons métalliques selon la revendication 3, **caractérisée en ce que** le Stéarate de Mg est employé.

5. Utilisation de savons selon l'une des revendications 1 à 4, **caractérisée en ce que** les savons sont utilisés de 0,5 à 3,5% (p/p), de préférence de 1 à 3% (p/p), de préférence de 1,5 à 2,5% (p/p) et d'une manière préférée entre toutes à environ 2% (p/p) sur la base du poids de la forme posologique.

6. Utilisation de savons selon l'une des revendications 1 à 5, **caractérisée en ce que** la forme posologique matricielle est produite par extrusion.

7. Utilisation de savons selon l'une des revendications 1 à 6, **caractérisée en ce que** la matrice comprend un polymère formant une matrice, au moins un composé pharmaceutiquement actif, un savon et facultativement des excipients pharmaceutiquement acceptables.

8. Utilisation de savons selon l'une des revendications 1 à 7, **caractérisée en ce que** la matrice est une matrice de diffusion.

9. Utilisation de savons selon la revendication 8, **caractérisée en ce que** le polymère formant une matrice est un polysaccharide, de préférence du groupe comprenant la cellulose, des dérivés de la cellulose ou l'amidon.

10. Utilisation de savons selon la revendication 9, **caractérisée en ce que** le polymère formant une matrice est de l'amidon, de préférence de l'amidon de pomme de terre, de tapioca ou de maïs.

11. Procédé pour augmenter la vitesse de libération des composés pharmaceutiquement actifs à partir des formes posologiques pharmaceutiques matricielles, ladite vitesse de libération est mesurée selon la méthode de la palette de la Pharm. Eur., **caractérisée en ce que** la forme posologique pharmaceutique est formulée avec au moins un composant de savon fournissant une libération accrue et/ou rapide de composés pharmaceutiquement actifs à partir des formes posologiques matricielles.

12. Procédé selon la revendication 11, **caractérisée en ce que** des savons selon l'une des revendications 1 à 10 sont employés.

13. Forme posologique pharmaceutique matricielle, **caractérisée en ce que** la forme posologique comprend au moins un savon pour fournir une libération accrue et/ou rapide d'un composé pharmaceutiquement actif à partir de la forme posologique en une quantité suffisante pour assurer une libération accrue et/ou rapide d'un composé pharmaceutiquement actif à partir de la forme posologique.

14. Forme posologique pharmaceutique matricielle selon la revendication 13, **caractérisée en ce que** au moins un savon alcalin, de préférence un ou plusieurs des sels alcalins d'acides gras supérieurs et de préférence les sels de Stéarate, Palmitate, Oléate ou Linoléate de Li, Na, K sont employés.

15. Forme posologique pharmaceutique matricielle selon la revendication 13, **caractérisée en ce que** au moins un savon métallique, de préférence un ou plusieurs sels métalliques d'acides gras supérieurs et de préférence les sels de Stéarate, Palmitate, Oléate ou Linoléate de Al, Ba, Ca, Cd, Co, Cr, Cu, Fe, Li, Mg, Mn, Ni, Pb, Sn, Sr, Zn sont employés.

16. Forme posologique pharmaceutique matricielle selon la revendication 15, **caractérisée en ce que** le Stéarate de Mg est employé en tant que savon métallique.

17. Forme posologique pharmaceutique matricielle selon l'une des revendications 13 à 16, **caractérisée en ce que** le savon est employé de 0,5 à 3,5% (p/p), de préférence de 1 à 3% (p/p), de préférence de 1,5 à 2,5% (p/p) et d'une manière préférée entre toutes à environ 2% (p/p) sur la base du poids de la forme posologique.

18. Forme posologique pharmaceutique matricielle selon l'une des revendications 13 à 17, **caractérisée en ce que** la forme posologique matricielle est produite par extrusion.

19. Forme posologique pharmaceutique matricielle selon l'une des revendications 13 à 18, **caractérisée en ce que** la matrice comprend un polymère formant une matrice, un composé pharmaceutiquement actif, un savon et facultativement des excipients pharmaceutiquement acceptables.

20. Forme posologique pharmaceutique matricielle selon l'une des revendications 13 à 19, **caractérisée en ce que** la matrice est une matrice de diffusion.

21. Forme posologique pharmaceutique matricielle selon l'une des revendications 13 à 20, **caractérisée en ce que** le polymère formant une matrice est un polysaccharide choisi dans le groupe comprenant la cellulose, les dérivés de la cellulose et l'amidon.

22. Forme posologique pharmaceutique matricielle selon la revendication 21, **caractérisée en ce que** le polymère formant une matrice est de l'amidon, de préférence de l'amidon de pomme de terre, de tapioca ou de maïs.
